(19) Europäisches Patentamt / European Patent Office / Office européen des brevets

(11) **EP 3 208 613 B1**

(12) **EUROPEAN PATENT SPECIFICATION**

(45) Date of publication and mention
of the grant of the patent:
**17.07.2019   Bulletin 2019/29**

(51) Int Cl.:
*G01N 33/52* *(2006.01)*      *C12Q 1/00* *(2006.01)*
*G01N 33/49* *(2006.01)*

(21) Application number: **15850564.4**

(22) Date of filing: **13.10.2015**

(86) International application number:
**PCT/CN2015/091793**

(87) International publication number:
**WO 2016/058511 (21.04.2016 Gazette 2016/16)**

(54) **SERUM FUCOSIDASE DETECTION KIT**

KIT ZUM NACHWEIS VON SERUMFUCOSIDASE

KIT DE DÉTECTION DE FUCOSIDASE DE SÉRUM

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.10.2014   CN 201410543027**

(43) Date of publication of application:
**23.08.2017   Bulletin 2017/34**

(73) Proprietor: **Medical System Biotechnology Co., Ltd.**
**Ningbo, Zhejiang 315104 (CN)**

(72) Inventors:
• **ZOU, Bingde**
  **Ningbo**
  **Zhejiang 315104 (CN)**
• **ZOU, Jihua**
  **Ningbo**
  **Zhejiang 315104 (CN)**
• **LI, Peng**
  **Ningbo**
  **Zhejiang 315104 (CN)**
• **JIA, Jianghua**
  **Ningbo**
  **Zhejiang 315104 (CN)**

(74) Representative: **Patentanwälte Gierlich & Pischitzis**
**Partnerschaft mbB**
**Gerbermühlstraße 11**
**60594 Frankfurt am Main (DE)**

(56) References cited:
CN-A- 1 731 146          CN-A- 1 731 146
CN-A- 101 003 831     CN-A- 101 169 368
CN-A- 104 267 178     US-A1- 2011 065 758
US-A1- 2014 134 606

• HUIMIN ZHAO ET AL: "Regeneration of cofactors for use in biocatalysis", CURRENT OPINION IN BIOTECHNOLOGY., vol. 14, no. 6, 1 December 2003 (2003-12-01), pages 583-589, XP055449232, GB ISSN: 0958-1669, DOI: 10.1016/j.copbio.2003.09.007

## Description

## Technical Field

The present disclosure relates to the field of medical testing and measuring technology, and in particular relates to a serum fucosidase detection kit.

$\alpha$-L-Fucosidase (AFU), is a lysosomal acid hydrolase, widely distributed in various tissues, cells, and bodily fluids of human bodies, and with comparatively higher tissue activity in the liver, kidney and etc. AFU as a lysosomal acid hydrolase, hydrolyzes lipid, mucin and mucopolysaccharides that contains fucose. Due to this characteristic, AFU reagent can be used as the activity detection of AFU. In 1977, Yves Deugnier et al. observed in animal experiments that the activity level of AFU in the liver cancer tissue of rat Morris was 7 times higher than normal liver, and long-term correlated with tumor. In 1984, Yves Deugnier et al. discovered elevated AFU in the serum of hepatocellular carcinoma patients, and the AFU was proposed to be used as a new enzyme to the diagnosis of liver cancer. The correlation of liver cancer and the AFU activity was confirmed by subsequent studies. In recent years, with further research of AFU detection method and AFU level variation in the serum of neoplastic or non-neoplastic disease, the determination of fucosidase activity combined with other laboratory indicia, has become a basic diagnostic index for liver cancer, gastric cancer, pancreatic cancer, colon cancer, oral cancer, leukemia, ovarian tumors, and etc. It has provided a huge improvement of the accuracy of diagnosis of related diseases and prognosis effect evaluation.

AFU detection kit has been widely used in the diagnosis of cancer, especially in the early detection of the primary liver cancer. The complementary advantages of joint detection of AFU index and AFP index has provided a positive detection rate of approx. 95%, facilitating the diagnosis of primary liver cancer, judgment of condition change and observation of treatment effect. Besides, AFU reagent has also been used as an important or supplementary index for diseases diagnosis and detection such as stomach cancer, pancreatic cancer, colon cancer, the oral cancer, leukemia, ovarian tumor and etc. There is a great potential for the AFU activity detection to be developed as a diagnostic index of large market capacity and wide application.

The main ways of AFU activity determination currently include fluorescence method, end-point colorimetric method and rate method.

(1) Fluorescence method: after the hydrolysis of fucosidase in the fucosidase compounds, 4-Methylumbelliferyl is released to emit fluorescence; the activity of enzyme is analyzed by examining the intensity of fluorescence. With high sensitivity, the method can be adopted on the serum, and a small amount of sample biopsy tissue, cells, cerebrospinal fluid and etc. However, the method requires high standard of the instruments, such as the fluorescence spectrophotometer and it is necessary to remove interfering substances by gel filtration, and automated analysis can't be achieved, thus, difficult to be popularized.

(2) End-point colorimetric method: PNP-AFU as a substrate, the substrate is enzymatically decomposed to produce a chromophore, and the chromophore exhibits a yellow color under alkaline conditions, then a quantitative analysis by spectrophotometric method. This method is simple and convenient and with no strict requirement of equipment. However, due to the poor anti-interference of the enzyme substrate, the influence of serum jaundice, hemolysis, lipemia and other factors on the measurement result can't be eliminated. The operation is complicated and a long reaction time is required. AFU as enzyme requires an acid environment for enzymatic reaction, while the chromophore requires an alkaline condition to exhibit color, thus, not suitable for automatic detection, and difficult to be popularized.

(3) Rate method: with AFU under suitable reaction condition to catalyze a hydrolysis of the specific substrate containing color group, the absorbance can be increased at a certain wavelength, and the continuous monitoring of the change of the absorbance, the AFU activity of the sample can be calculated. It is a simple operation, with short determination time, and the sensitivity and anti-interference are comparatively improved, suitable for various half/full automatic analysis instruments and for clinical popularization and application.

Since the rate method has the advantages for clinical popularization and application, the AFU activity tests in clinical medicine has been applied widely in recent years, however, the substrate used in the method is generally unstable, and the sensitivity should also be further improved. The present disclose provides a high-performance serum fucosidase detection kit, to solve the stability and sensitivity problems of the substrate.

## Description

To solve the technical problems of the prior art, the present disclosure provides a serum fucosidase detection kit with stable and high-sensitivity substrate to guarantee an accurate and reliable measurement.

The technical scheme of the present disclosure is as follows: a serum fucosidase detection kit, comprises a reagent 1 and a reagent 2,

| reagent 1: | buffer (pH4.0-4.5) | 50 -500 mmol/l |
| | surface active agent | 1 -50 g/l |
| | anti-interference agent | 1-20 g/L |
| | preservative | 0.1 -100 g/L; |
| reagent 2: | buffer (pH7.0-7.5) | 50 -500 mmol/L |
| | glucose | 2-60 g/L |
| | hexokinase | 1 -20 KU/L |
| | glucose-6-phosphate dehydrogenase | 1 -20 KU/l |
| | magnesium sulfate | 0.3-5 g/L |
| | substrate | 0.2-5 g/L |
| | stabilizer | 1-100 mmol/L |
| | protective agent | 0.1-40 g/L |
| | preservative | 0.1-100 g/L, |

wherein the substrate is 2-chloro-p-nitrophenol-$\alpha$-L-fucoside (CNP-AFU) or polyvinylsulfuric acid potassium salt with CNP-AFU and the stabilizer is reduced coenzyme I (NADH) or reduced coenzyme II (NADPH).

The buffer of the present disclosure is one or more of tris-hydroxymethyl amino buffer, glycine-NaOH buffer, citric acid-sodium citrate buffer, disodium hydrogen phosphate-citric acid buffer, acetic acid-sodium acetate buffer, MES buffer, Good's buffer and borate buffer.

The pH of the reagent 1 is at 4.0 -4.5 pH and reagent 2 at 7.0 -7.5. A double-reagent can effectively improve the anti-interference performance, and protect the stability of the substrate in reagent 2 by the enzyme cycling protection systems.

The surfactant of the present disclosure is a nonionic surfactant, preferably one or more of Tween-20, Triton 100, Brij35 and etc. One or more of the above nonionic surfactants are found by the experiment and analysis to be the most optimal; in addition, this choice of surfactants has a good result for improving the sensitivity of the reaction.

The preservative of the present disclosure is one or more of G418 (CAS No.:108321-42-2, molecular formula: $C_{20}H_{40}N_4O_{10} \cdot 2(H_2SO_4)$, molecular weight: 692.71), kanamycin, sodium azide, and Proclin300. The selection of the preservative is to prevent the growth of bacteria and the bacterial destruction of raw materials in the kit.

The anti-interference agent of the present disclosure is bilirubin oxidase. This anti-interference agent is to prevent the interference of bilirubin.

The substrate of the present invention is 2-chloro-p-nitrophenol-$\alpha$-L-fucoside (2-chloro-4-nitrophenyl-$\alpha$-L-fucoside CNP-AFU) or M-G-CNP-AFU (Shanghai Seebio Biotech, Inc. Cat# 140205, Chinese name: M-G-CNP-AFU substrate, polyvinylsulfuric acid potassium salt with CNP-AFU; molecular formula $CH_2CH(OSO_3K)]_n$ $C_{12}H_{14}NO_7Cl$, molecular weight 3542.6). The selection of the substrate is related to the sensitivity of the kit and the stability of the substrate. The stability of these two substrates is relatively higher than the others.

The stabilizer of the present invention is reduced coenzyme I (NADH) or reduced coenzyme II (NADPH). This stabilizer combines with the enzymes in the reagent 2 constitutes an enzymatic cycling system, to keep the stability of the substrates, and it does not participate in the reaction process of detection.

The protective agent of the present disclosure is one or more of polyethylene glycol, glycerol, propylene glycol, sucrose, trehalose, sorbitol and BSA. The protective agent herein primarily protects the several raw materials of enzyme in the reagent 2 to keep the stability of the kit.

The preparing method of the reagent 1 and the reagent 2 of the present disclosure is to respectively mix the components according to the formula ratio requirements with distilled water and stir evenly.

The test conditions of AFU in the sample of the present disclosure includes: temperature: 30°C to 37°C; the light path of the cuvette is 1.0 cm; the dominant wavelength of detection is 405 nm and the secondary wavelength 480 nm.

The method of the present disclosure for determining AFU in the sample is as follows: the sample (use a calibration tube for a calibrator as the sample) is added and uniformly mixed with R1, 30 to 37°C incubation for 3-5 minutes, then added and uniformly mixed with R2, 30 to 37°C reaction for 90 seconds, monitor the change of the absorbance for 90 seconds. Wherein the amount of the sample is 10-25 $\mu$l, the reagent 1 is 240 $\mu$l, and the reagent 2 is 80 $\mu$l.

The content of AFU activity in the sample of the present disclosure is calculated by the following equation:

$$AFU\ Activity\ (U/L) = \frac{\triangle A/min_{Sample} - \triangle A/min_{Blank}}{\triangle A/min_{Standard} - \triangle A/min_{Blank}} \times C_{Standard}$$

Advantages and beneficial effects of the present disclosure include:

1. the present disclosure has solved the problem of instability of the substrate in the AFU detection method, by adding stabilizer, that is, reduced coenzyme I(NADH) or reduced coenzyme II (NADPH) to protect the substrate; and meanwhile, the enzyme cycling protection system of hexokinase and glucose-6-phosphate dehydrogenase, eliminates the unstable factors caused by the degradation of NADH or NADPH and ensures the protection of the substrate by the stabilizer.

2. the dual reagent formulated in the method adopted in the present disclosure is stable for at least 12 months at 2-8°C, and can be used directly without re-dissolution. The method is simple, easy to operate, and a detection result can be quickly obtained.

**Brief Description of the Drawings**

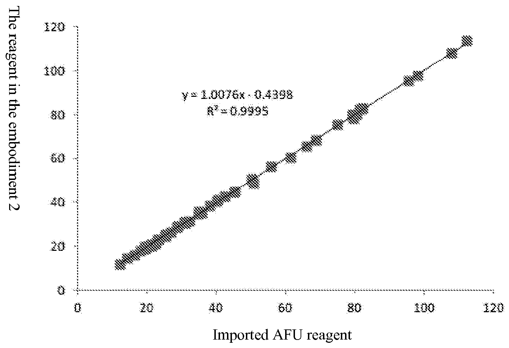

The correlation curve in the drawing is an illustration of the reference data in table 2 in the embodiment.

**Embodiment**

The present disclosure will be further illustrated by the following non-limiting embodiments, well known to those skilled in the art. Without departing from the spirit of the disclosure, it is possible to make many modifications of the present disclosure; such modifications are also intended to fall within the protection scope of the present disclosure.
The following experimental methods unless otherwise indicated, are considered as conventional methods, the experimental materials selected unless otherwise indicated, can all be readily obtained from commercial companies.

Embodiment 1

| | | |
|---|---|---|
| reagent 1: | acetic acid-sodium acetate buffer (pH4.5) | 100 mmol/l |
| | Tween 20 | 15 g/L |
| | bilirubin oxidase | 2 KU/L |
| | G418 | 10 g/L |
| reagent 2: | Good 's buffer (pH7.0) | 100 mmol/L |
| | glucose | 20 g/L |
| | hexokinase | 5 KU/L |
| | glucose-6-phosphate dehydrogenase | 4 KU/l |
| | magnesium sulfate | 1 g/L |
| | CNP-AFU | 2 g/L |
| | NADH | 12 mmol/L |
| | polyethylene glycol 6000 | 6 g/L |
| | G418 | 1 g/L |

The preparing method of the reagent 1 and the reagent 2 is conventional, that is, to respectively mix the components according to the formula ratio requirements with distilled water and stir evenly.

Embodiment 2

| | | |
|---|---|---|
| reagent 1: | MES buffer (pH4.5) | 300 mmol/L |
| | Triton 100 | 40 g/L |

(continued)

| | | |
|---|---|---|
| | bilirubin oxidase | 4 KU/L |
| | Proclin 300 | 3 g/L |
| reagent 2: | Good 's buffer (pH7.5) | 200 mmol/L |
| | glucose | 50 g/L |
| | hexokinase | 15 KU/L |
| | glucose-6-phosphate dehydrogenase | 10 KU/L |
| | magnesium sulfate | 3 g/L |
| | M-G-CNP-AFU | 4 g/L |
| | NADPH | 80 mmol/L |
| | sucrose | 40 g/L |
| | sodium azide | 2 g/L |

The preparing method of the embodiment 2 is the same as of the embodiment 1.

Embodiment 3 for comparison

| | | |
|---|---|---|
| reagent 1: | MES buffer (pH4.5) | 300 mmol/L |
| | Triton 100 | 40 g/L |
| | bilirubin oxidase | 4 KU/L |
| | Proclin 300 | 3 g/L |
| Reagent 2: | Good 's buffer (pH7.5) | 200 mmol/L |
| | M-G-CNP-AFU | 4 g/L |
| | Sucrose | 40 g/L |
| | sodium azide | 2 g/L |

The preparing method of the embodiment 3 is the same as of the embodiment 2, except for the removal of protection system for the substrate.

Here is the illustration of the comparison of performance of embodiment 2 and embodiment 3 in conjunction with the tables and drawing.

1. the stability of the substrate

37°C accelerated stability experiments (to formulate the reagent for 37°C accelerated stability test, and an accelerated stability test at 37°C for 7 days can simulate the situation of a real storage at 4-8°C for 1 year) indicate that the kit of embodiment 2 is of good stability; there is no degradation of the substrate and limited rise of the blank absorbance; while a significant rise of the reagent blank in the kit of embodiment 3. The substrate degradation is caused due to the absence of the protection system, which further leads to a significant rise in the blank. The results are shown in table 1:

Table 1 detection data of the absorbance of the reagent blank in embodiment 2 and embodiment 3

| Time | Embodiment 2 Absorbance of the reagent blank | Embodiment 3 Absorbance of the reagent blank |
|---|---|---|
| The 1st day | 0.0243 | 0.0251 |
| 37°C for 3 days | 0.0246 | 0.1384 |
| 37°C for 5 days | 0.0257 | 0.2458 |
| 37°C for 7 days | 0.0264 | 0.3127 |
| Rising percentage on the 7th day | 8.6% | 1145.8% |

2. comparative experiments methodology

The measurement data of the detection kit of embodiment 2 and one imported AFU reagent applied in 50 cases of clinical sample serum are shown in table 2:

Table 2: correlation study results of the reagent in embodiment 2 of the present disclosure and an imported AFU reagent

| Number | Reagent of embodiment 2 | Imported AFU reagent |
| --- | --- | --- |
| 1 | 14.46 | 14.65 |
| 2 | 12.32 | 12.15 |
| 3 | 19.28 | 19.09 |
| 4 | 45.26 | 44.6 |
| 5 | 19.38 | 18.89 |
| 6 | 31 | 30.59 |
| 7 | 80.57 | 80.32 |
| 8 | 30.77 | 31.1 |
| 9 | 35.92 | 35.26 |
| 10 | 21.52 | 20.61 |
| 11 | 25.64 | 24.69 |
| 12 | 50.24 | 50.67 |
| 13 | 25.49 | 25.33 |
| 14 | 23.6 | 23.32 |
| 15 | 82.31 | 82.82 |
| 16 | 98.12 | 98.04 |
| 17 | 27.08 | 26.41 |
| 18 | 32.16 | 31.4 |
| 19 | 75.02 | 75.66 |
| 20 | 29.02 | 29.02 |
| 21 | 79.81 | 78.39 |
| 22 | 79.48 | 80.12 |
| 23 | 19.48 | 19.78 |
| 24 | 45.42 | 45.05 |
| 25 | 19.58 | 19.39 |
| 26 | 50.8 | 48.98 |
| 27 | 20.77 | 20.43 |
| 28 | 21.36 | 20.18 |
| 29 | 66.23 | 65.88 |
| 30 | 61.31 | 60.53 |
| 31 | 25.16 | 25.18 |
| 32 | 40.33 | 40.59 |
| 33 | 34.92 | 34.89 |
| 34 | 23.19 | 23.15 |
| 35 | 81.56 | 82.61 |

(continued)

| Number | Reagent of embodiment 2 | Imported AFU reagent |
|--------|-------------------------|----------------------|
| 36 | 38.08 | 38.4 |
| 37 | 95.51 | 95.63 |
| 38 | 22.45 | 21.42 |
| 39 | 35 | 35.74 |
| 40 | 107.83 | 108.25 |
| 41 | 28.62 | 29 |
| 42 | 19.88 | 20.03 |
| 43 | 68.74 | 68.55 |
| 44 | 16.41 | 16.35 |
| 45 | 18.22 | 17.99 |
| 46 | 40.56 | 41.33 |
| 47 | 42.68 | 43.01 |
| 48 | 55.86 | 56.21 |
| 49 | 79.52 | 79.63 |
| 50 | 112.35 | 113.86 |

A correlation curve has been made in the drawing according to the above data, as the drawing shows, the data fits well, and has provided sufficient proof that the kit of the present disclosure has reached an advanced level in the world. The substrate is stable and can be used directly without re-dissolution; the method is simple, easy to operate, and a detection result can be quickly obtained.

**Claims**

1.  A serum fucosidase detection kit, comprises a reagent 1 and a reagent 2,

| | | |
|---|---|---|
| reagent 1: | buffer (pH4.0-4.5) | 50 -500 mmol/l |
| | surface active agent | 1 -50 g/l |
| | anti-interference agent | 1-20 g/L |
| | preservative | 0.1 -100 g/L; |
| reagent 2: | buffer (pH7.0-7.5) | 50 -500 mmol/L |
| | glucose | 2-60 g/L |
| | hexokinase | 1 -20 KU/L |
| | glucose-6-phosphate dehydrogenase | 1 -20 KU/l |
| | magnesium sulfate | 0.3-5 g/L |
| | substrate | 0.2-5 g/L |
| | stabilizer | 1-100 mmol/L |
| | protective agent | 0.1-40 g/L |
| | preservative | 0.1-100 g/L, |

wherein the substrate is 2-chloro-p-nitrophenol-$\alpha$-L-fucoside (CNP-AFU) or polyvinylsulfuric acid potassium salt with CNP-AFU and the stabilizer is reduced coenzyme I (NADH) or reduced coenzyme II (NADPH).

2.  The serum fucosidase detection kit of claim 1, wherein the buffer is one or more of tris-hydroxymethyl amino buffer, glycine-NaOH buffer, citric acid-sodium citrate buffer, disodium hydrogen phosphate-citric acid buffer, acetic acid-sodium acetate buffer, MES buffer, Good's buffer and borate buffer.

3. The serum fucosidase detection kit of claim 1, wherein the surfactant is a nonionic surfactant.

4. The serum fucosidase detection kit of claim 3, wherein the nonionic surfactant is one or more of Tween-20, Triton 100 and Brij35.

5. The serum fucosidase detection kit of claim 1, wherein the preservative is one or more of G418, kanamycin, sodium azide, and Proclin300.

6. The serum fucosidase detection kit of claim 1, wherein the anti-interference agent is bilirubin oxidase.

7. The serum fucosidase detection kit of claim 1, wherein the protective agent is one or more of polyethylene glycol, glycerol, propylene glycol, sucrose, trehalose, sorbitol and BSA.

**Patentansprüche**

1. Serum-Fucosidase-Nachweiskit umfasst ein Reagenz 1 und ein

   Reagenz 2, Reagenz 1: Puffer (pH 4,0-4,5) 50-500 mmol/l
   oberflächenaktives Mittel 1- 50 g/l
   Anti-Interferenzmittel 1-20 g/l
   Konservierungsmittel 0,1-100 g/l;
   Reagenz 2: Puffer (pH 7,0-7,5) 50-500 mmol/l
   Glucose 2-60 g/l
   Hexokinase 1 - 20 KU/l
   Glucose-6-phosphat-Dehydrogenase 1 -20 KU/l
   Magnesiumsulfat 0,3-5 g/l
   Substrat 0,2-5 g/l
   Stabilisator 1-100 mmol/l
   Schutzmittel 0,1-40 g/l
   Konservierungsmittel 0,1-100 g/l,
   wobei das Substrat 2-Chlor-p-nitrophenol-$\alpha$-L-Fucosid (CNP-AFU) oder Polyvinylschwefelsäure-Kaliumsalz mit CNP-AFU ist und wobei der Stabilisator reduziertes Coenzym I (NADH) oder reduziertes Coenzym II (NADPH) ist.

2. Serum-Fucosidase-Nachweiskit nach Anspruch 1, wobei der Puffer einer oder mehrere der folgenden Stoffe ist: Tris-Hydroxymethyl-Aminopuffer, Glycin-NaOH-Puffer, Zitronensäure-Natriumcitrat-Puffer, Dinatriumhydrogenphosphat-Zitronensäure-Puffer, Essigsäure-Natriumacetat-Puffer. MES-Puffer, Good-Puffer und Boratpuffer.

3. Serum - Fucosidase - Nachweiskit nach Anspruch 1, wobei das Tensid ein nichtionisches Tensid ist.

4. Serum-Fucosidase-Nachweiskit nach Anspruch 3, wobei das nichtionische Tensid eines oder mehrere von Tween-20, Triton 100 und Brij35 ist.

5. Serum-Fucosidase-Nachweiskit nach Anspruch 1, wobei das Konservierungsmittel eines oder mehrere von G418, Kanamycin, Natriumazid und Proclin300 ist.

6. Serum-Fucosidase-Nachweiskit nach Anspruch 1, wobei das Anti-Interferenzmittel Bilirubinoxidase ist.

7. Serumfucosidase-Nachweiskit nach Anspruch 1, wobei das Schutzmittel eines oder mehrere von Polyethylenglykol, Glycerin, Propylenglykol, Saccharose, Trehalose, Sorbit und BSA ist.

**Revendications**

1. Kit de détection de la fucosidase sérique comprend un réactif 1 et un

   réactif 2, réactif 1: tampon (pH 4,0 - 4,5), 50 -500 mmol/l.

agent de surface 1 -50 g/l

agent anti-ingérence 1-20 g/L

agent de conservation 0,1 -100 g/L;

réactif 2: tampon (pH 7,0 -7,5) 50 à 500 mmol/L

glucose 2-60 g/l

hexokinase 1 -20 KU/L

glucose-6-phosphate déshydrogénase 1 -20 KU/l

sulfate de magnésium 0,3-5 g/L

substrat 0,2-5 g/L

stabilisateur 1-100 mmol/L

agent de protection 0,1-40 g/L

agent de conservation 0,1-100 g/L,

dans lequel le substrat est le sel de potassium de l'acide 2-chloro-p-nitrophénol-$\alpha$-L-fucoside (CNP-AFU) ou de l'acide polyvinylsulfurique avec CNP-AFU et le stabilisant est la coenzyme I réduite (NADH) ou réduite en coenzyme II (NADPH).

2. Kit de détection de fucosidase sérique selon la revendication 1, dans lequel le tampon est un ou plusieurs des tampons tris-hydroxyméthylamino, tampon glycine-NaOH, tampon acide citrique-citrate de sodium, tampon disodiumhydrogénophosphate-acide citrique, tampon acide acétique-acétate de sodium, Tampon MES, tampon de Good et tampon de borate.

3. Kit de détection de fucosidase sérique selon la revendication 1, dans lequel le tensioactif est un tensioactif non ionique.

4. Kit de détection de fucosidase sérique selon la revendication 3, dans lequel le tensioactif non ionique est un ou plusieurs parmi Tween-20, Triton 100 et Brij35.

5. Kit de détection de fucosidase sérique selon la revendication 1, dans lequel le conservateur est un ou plusieurs parmi G418, la kanamycine, l'azide de sodium et Proclin300.

6. Kit de détection de fucosidase sérique selon la revendication 1, dans lequel l'agent anti-interférence est la bilirubine oxydase.

7. Kit de détection de fucosidase sérique selon la revendication 1, dans lequel l'agent protecteur est un ou plusieurs parmi le polyéthylène glycol, le glycérol, le propylène glycol, le saccharose, le tréhalose, le sorbitol et le BSA.